# EUROPEAN PATENT SPECIFICATION

(11) **EP 1 919 532 B1**
(45) Date of publication and mention of the grant of the patent: **20.07.2016**
(21) Application number: 06784448.0
(22) Date of filing: 22.05.2006
(51) Int. Cl.: A61L 31/02, A61L 31/10, A61L 31/14, A61F 2/82

(54) **BIOABSORBABLE STENT**
BIORESORBIERBARER STENT
ENDOPROTHESE BIORESORBABLE

(30) Priority: 30.08.2005 US 213817
(43) Date of publication of application: 14.05.2008
(73) Proprietor: Boston Scientific Limited, Hamilton HM11 (BM)
(72) Inventor: FLANAGAN, Aiden, Co. Galway (IE)
(74) Representative: Peterreins Schley
(86) International application number: PCT/US2006/019784
(87) International publication number: WO 2007/027251

(56) References cited:
- WO-A1-2007/005806
- WO-A2-2006/108065
- DE-A1-102005 018 356
- US-A1- 2003 033 007
- US-A1- 2004 034 409

## Description

### Background

The disclosed invention relates generally to a bioabsorbable stent.

Intraluminal stents are typically inserted or implanted into a body lumen, for example, a coronary artery, after a procedure such as percutaneous transluminal coronary angioplasty. Such stents are used to maintain the patency of a body lumen by supporting the walls of the lumen and preventing abrupt reclosure or collapse thereof. These stents can also be provided with one or more therapeutic agents adapted to be locally released from the stent at the site of implantation. In the case of a coronary stent, the stent can be adapted to provide release of, for example, an antithrombotic agent to inhibit clotting or an antiproliferative agent to inhibit smooth muscle cell proliferation, i.e., neointimal hyperplasia, which is believed to be a significant factor leading to re-narrowing or restenosis of the blood vessel after implantation of the stent.

Stents are commonly formed from biocompatible metals such as stainless steel, or metal alloys such as nickel-titanium alloys that are often employed because of their desirable shape-memory characteristics. Metallic materials are advantageously employed to construct stents because of the inherent rigidity of metallic materials and the consequent ability of the metallic stent to maintain patency of the lumen upon implantation of the stent. Metallic stents can also cause complications, however, such as thrombosis and neointimal hyperplasia. It is believed that prolonged contact of the metallic surfaces of the stent with the lumen may be a significant factor in these adverse events following implantation.

The above described potential adverse affects of metallic stents can be reduced by adapting the stent to provide localized release of a therapeutic agent. To provide the therapeutic agent, metallic stents are coated with a biodegradable or non-biodegradable material containing the therapeutic agent. The coating may also provide a more biocompatible surface directly in contact with the body lumen wall.

US2004/034409 discloses a stent comprising a structure, formed preferably of a magnesium alloy and a coating of poly-L-lactide. The lactide coating can serve as reservoir for pharmacologically active drugs. For a better adhesion, a passive silicon carbide passive coating can be applied on the metal body. The implant should be covered with the polymeric coating over as large a surface area as possible.

The use of such drug eluting stents has helped to reduce the occurrence of restenosis of the body lumen; however, physicians would prefer not to leave the stent permanently in the body lumen. When a body lumen requires multiple stenting procedures, complications for later surgeries can result, creating what is commonly referred to as a "full metal jacket." Because of this, biodegradable stents constructed of a magnesium alloy or biodegradable polymers have recently been developed. These magnesium stents can be absorbed by the body over time.

Although the use of stents formed with magnesium alloys, iron alloys, or biodegradable polymers provides both functional and safety benefits over conventional non-absorbable stents, and the characteristic rate of bioabsorption of the materials used in such stents can lead to other problems. The absorption rate of the stent depends on a variety of factors including the material composition, the size and surface area of the stent, and physiology and biochemical interactions with the stent at the treatment site in the body lumen. Rapid bioabsorption of bioabsorbable stents may be undesirable for both health and functional reasons. For example, rapid bioabsorption of bioabsorbable stents may result in the stent being absorbed before the body lumen has sufficiently healed and become self-supporting. Further, high rates of absorption of the stents' constituent materials may have undesirable physiological effects. Conversely, if the stent is absorbed too slowly, it may interfere with a subsequent stenting procedure. Thus, there is a need for a bioabsorbable stent that can be configured with a preselected and controllable rate of absorption.

### Summary of the Invention

The invention relates to a stent (20), comprising a support structure (22) formed of a first material, said first material being a metal that is absorbable by a mammalian body; and an absorption inhibitor (24) disposed on said support structure (22) in at least partially overlying relationship, said absorption inhibitor (24) being formed of a second material different from said first material, said second material being non-absorbable by the mammalian body, said absorption inhibitor (24) reducing a rate of absorption of said portion of said support structure (22) underlying said absorption inhibitor (24), wherein the support structure (22) has an inner surface (28) and an outer surface (30) and defines a lumen (26) characterized in that the absorption inhibitor (24) is alternatively disposed on the outer surface (30) or on the inner surface (28), wherein there is varying thickness of the absorption inhibitor (24) on a particular portion of the support structure (22).

### Brief Description of the Drawings

The present invention is described with reference to the accompanying drawings. In the drawings, like reference numbers indicate identical or functionally similar elements. For example, item 20 is identical or functionally similar to item 120.
FIG. 1 is an illustration of a stent according to an embodiment of the invention shown positioned in a cross-sectional view of a body lumen.
FIG. 2 is an end view of the stent illustrated in FIG. 1.
FIG. 3 is an alternative end view of the stent illustrated in FIG. 1.
FIG. 4 is a side perspective view of a stent according to an embodiment of the invention.
FIG. 5 is a side perspective view of a stent according to another embodiment of the invention shown positioned in a cut-away view of a portion of blood vessel.
FIG. 6 is a side perspective view of a stent according to another embodiment of the invention.
FIG. 7 is a side perspective view of a stent according to another embodiment of the invention.
FIG. 8 is a sectional view taken along line 8-8 in FIG. 7.
FIG. 9 is a sectional view taken along line 9-9 in FIG. 7.
FIG. 10 is a side perspective view of a stent according to another embodiment of the invention.
FIGS. 11, 12 and 13 are a side perspective views of a stent according to another embodiment of the invention shown positioned in a cross-sectional view of a body lumen at various stages of a bioabsorption process.

### Detailed Description

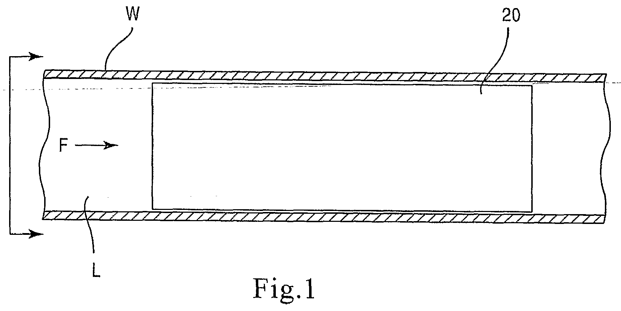

FIG. 1 is a schematic illustration of a stent 20 embodying the principles of the invention. The stent 20 is configured to be placed or otherwise implanted into a natural body lumen L of a mammal (e.g., a blood vessel or ureter) to support the walls W of the body lumen L, such as after a medical procedure (e.g., a coronary angioplasty). A bodily fluid F may flow through lumen L. The stent 20 is constructed of materials that are configured to be absorbed by the mammalian body over a controlled or predetermined period of time.

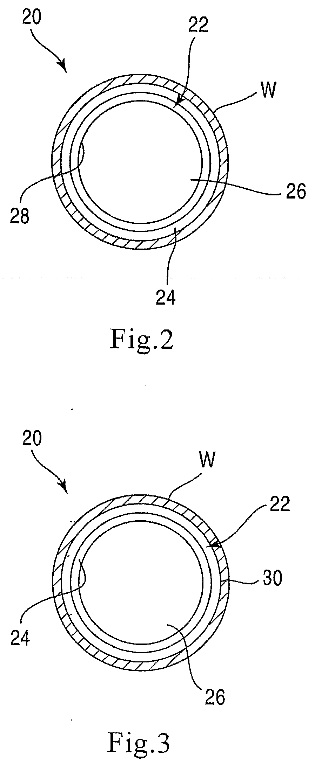

Specifically, the stent 20 includes a support structure 22 and an absorption inhibitor layer 24. FIGS. 2 and 3 illustrate two alternative cross-sections of the stent 20 shown in FIG. 1. The support structure 22 includes an inner surface 28 and an outer surface 30, and defines a lumen 26. FIG. 2 illustrates stent 20 having an absorption inhibitor layer 24 disposed on the outer surface 30 of stent 20. FIG. 3 illustrates stent 20 having an absorption inhibitor layer 24 disposed on the inner surface 28 of stent 20. The support structure 22 may be constructed in a variety of different configurations, including any of the conventional configurations commonly used, such as a lattice or network of struts forming a framework having multiple apertures or open spaces, a perforated tube, or a coil configuration.

The support structure 22 is configured to provide the desired degree of structural support for the body lumen L. The desired degree of structural support decreases over time. For example, a stent disposed in a coronary artery after an angioplasty requires a certain degree of structural strength to resist relapse of the coronary artery immediately after the procedure. As the artery heals, less structural support is required to prevent relapse. Eventually no internal support is needed. After that point in time, it is desirable for the support structure to be absent from the artery, for the reasons described above. A support structure 22 formed of a bioabsorbable material can meet this time-varying support requirement, in that it can be configured such that before the onset of any biodegradation it provides the requisite initial structural support to the lumen L, and such that it is completely biodegraded after the time at which structural support to the body lumen L is no longer required and before the time by which it is desired that the artery be clear of the stent 20.

The structural strength of a stent as a function of time after placement in the artery may not meet the desired profile. For example, if the stent immediately begins to biodegrade, its structural strength may drop below the required value. It is undesirable to add more material to the stent so that it remains at or above the required strength at all points in time because, for example, this would increase the total amount of material absorbed by the body and during delivery, for example, when the stent is mounted on a balloon catheter, make the stent bulkier and/or less flexible and maneuverable. Therefore, the biodegradation of the support structure 22 of the stent 20 can be modulated or controlled by the presence of the absorption inhibitor layer 24.

The absorption inhibitor layer 24 can reduce the rate of absorption of the surface(s) of the support structure 22 that it overlies, and may reduce the rate of absorption to zero. If the absorption inhibitor layer 24 is not absorbed, its effect persists until the underlying surface of the support structure 22 is absorbed (through the absorption inhibitor layer 24 at a reduced rate and/or from another direction). Thus, the duration of the absorption inhibitor layer's effect on the rate of absorption of the underlying surface of the support structure 22 depends on its thickness.

By varying the thickness of the absorption inhibitor layer 24 on a particular portion of support structure 22, the absorption of some portions of the support structure 22 can be delayed longer than other portions. Thus, the stent 20 can be configured such that selected portions of the support structure 22 can remain in position in the body lumen for longer periods of time. Portions of the support structure 22 may have no absorption inhibitor layer 24. These portions of support structure 22 will begin to bioabsorb immediately upon implantation into the body lumen. Alternatively, the thickness of the absorption inhibitor layer 24 may be constant.

An absorption inhibitor layer 24 disposed on a radially outer surface of a cardiovascular stent would be in contact with the inner wall of the coronary artery, while an absorption inhibitor layer 24 disposed on the radially inner surface would be in contact with the blood flowing through the artery. The physiochemical properties of the artery will change as the artery heals following an angioplasty procedure. Delaying the absorption of some or all of the support structure 22 allows time for the support structure 22 to be encapsulated by the body lumen intima before starting to bioabsorb, which in turn can lead to a different rate of absorption of the support structure 22.

An absorption inhibitor layer 24 that is formulated to allow, at a reduced rate, absorption of the surface of the support structure 22 underlying the absorption inhibitor layer 24, can also have a time-varying effect on the absorption rate. For example, the absorption mechanism by which the support structure 22 is absorbed through the absorption inhibitor layer 24 can depend on the thickness of the absorption inhibitor layer 24 as described above. There are, therefore, several variables affecting the time profile of the absorption rate of the surface of the support structure 22 underlying the absorption inhibitor layer 24, including the thickness of the absorption inhibitor layer 24 and the degree to which it reduces the rate of absorption of the underlying support structure material, which in turn may vary with other factors such as thickness.

These factors provide significant flexibility in tailoring the time profile of the absorption rate of the surface of the support structure 22 underlying the absorption inhibitor layer 24. In turn, the integral of the absorption rate over time yields the amount of support structure 22 absorbed as a function of time. In combination with the other factors, such as the structural properties of the support structure material, the initial local 6 thickness, and support structure geometry/configuration, the amount of absorption over time determines the support structure's degree of support to the body lumen L, and the amount over time.

The spatial distribution of the absorption inhibitor layer 24 over the surfaces of the support structure 22, including thickness and location, can also be varied to yield the desired time profile of support and quantity of support structure material remaining. Thus, the absorption inhibitor layer 24 can be disposed on the entirety of the outer surface of a tubular support structure 22, and/or the entirety of the inner surface, and/or one or both end surfaces, and/or on the sides of perforated struts, links, or other such surfaces of the support structure 22. The absorption inhibitor layer 24 can also be placed on a portion, rather than the entirety, of any or all of the above surfaces. The portions of any surface can be a continuous portion (such as one-half of a surface), one or more discontinuous portions (in radial or circumferential strips (parallel and/or intersecting)), circular or polygonal patches or spots, etc. The thickness of the absorption inhibitor layer(s) 24 can be constant or can vary from surface to surface or along one surface.

The absorption inhibitor layer 24 can be homogeneous (i.e. uniform composition) or heterogeneous. Thus, different absorption inhibitor materials, with different properties, can be used in combination. The different materials can be used on different surfaces, on the same surfaces (spaced or abutting), or partially or completely overlapping.

Thus, the time by which the support structure 22 will absorb will depend on a number of factors identified above, including the shape and size of the support structure 22, and the particular composition of the alloy. The material of the support structure 22 may be configured and formulated such that the support structure 22 is substantially completely absorbed into the body in 14 to 56 days. The absorption of the support structure 22 may be delayed such that it is substantially completely absorbed by the body within 12 months after implantation into the body. The stent 20 may be configured to retain at least approximately 90% of its structural strength for at least 180 days.

Optionally, stent 20 can include a delivery layer 36, disposed on some or all surfaces of support structure 22, by which a therapeutic agent can be delivered to the body. Therapeutic agents are commonly used to help reduce restenosis and thrombosis of the body lumen. Delivery layer 36 can be of any conventional formulation or composition, to deliver any known therapeutic agent. Delivery layer 36 can be formulated to bioabsorb, delivering the therapeutic agent as it is absorbed, or not to bioabsorb, delivering the therapeutic agent by other mechanisms. In either case, absorption inhibitor layer 24 can be arranged to overlie delivery layer 36 to modulate the rate of relapse of the therapeutic agent in a manner similar to the way in which it can modulate the rate of absorption of the support structure 22. In other embodiments, the function of delivery layer 36 and absorption inhibitor layer 24 can be combined, i.e., the absorption inhibitor layer 24 can be formulated to include a therapeutic agent.

As used herein, the term "therapeutic agent" includes, but is not limited to, any therapeutic agent or active material, such as drugs, genetic materials, and biological materials. Suitable genetic materials include, but are not limited to, DNA or RNA, such as, without limitation, DNA/RNA encoding a useful protein, DNA/RNA intended to be inserted into a human body including viral vectors and non-viral vectors, and RNAi (RNA interfering sequences). Suitable viral vectors include, for example, adenoviruses, gutted adenoviruses, adeno-associated viruses, retroviruses, alpha viruses (Semliki Forest, Sindbis, etc.), lentiviruses, herpes simplex viruses, ex vivo modified and unmodified cells (*e.g.,* stem cells, fibroblasts, myoblasts, satellite cells, pericytes, cardiomyocytes, skeletal myocytes, macrophage), replication competent viruses (*e.g*., ONYX-015), and hybrid vectors. Suitable non-viral vectors include, for example, artificial chromosomes and mini-chromosomes, plasmid DNA vectors (*e.g*., pCOR), cationic polymers (*e.g*., polyethyleneimine, polyethyleneimine (PEI)) graft copolymers (*e.g*., polyether-PEI and polyethylene oxide-PEI), neutral polymers PVP, SP1017 (SUPRATEK), lipids or lipoplexes, nanoparticles and microparticles with and without targeting sequences such as the protein transduction domain (PTD).

Suitable biological materials include, but are not limited to, cells, yeasts, bacteria, proteins, peptides, cytokines, and hormones. Examples of suitable peptides and proteins include growth factors (*e.g*., FGF, FGF-1, FGF-2, VEGF, Endothelial Mitogenic Growth Factors, and epidermal growth factors, transforming growth factor α and β, platelet derived endothelial growth factor, platelet derived growth factor, tumor necrosis factor α, hepatocyte growth factor and insulin-like growth factor), transcription factors, proteinkinases, CDK inhibitors, thymidine kinase, and bone morphogenic proteins (BMP's), such as BMP-2, BMP-3, BMP-4, BMP-5, BMP-6 (Vgr-1), BMP-7 (OP-1), BMP-8. BMP-9, BMP-10, BMP-11, BMP-12, BMP-13, BMP-14, BMP-15, and BMP-16. These dimeric proteins can be provided as homodimers, heterodimers, or combinations thereof, alone or together with other molecules. Cells can be of human origin (autologous or allogeneic) or from an animal source (xenogeneic), genetically engineered, if desired, to deliver proteins of interest at a desired site. The delivery media can be formulated as needed to maintain cell function and viability. Cells include, for example, whole bone marrow, bone marrow derived mono-nuclear cells, progenitor cells (*e.g*., endothelial progentitor cells), stem cells (*e.g*., mesenchymal, hematopoietic, neuronal), pluripotent stem cells, fibroblasts, macrophage, and satellite cells.

The term "therapeutic agent" and similar terms also includes non-genetic agents, such as: anti-thrombogenic agents such as heparin, heparin derivatives, urokinase, and PPack (dextrophenylalanine proline arginine chloromethylketone); anti-proliferative agents such as enoxaprin, angiopeptin, or monoclonal antibodies capable of blocking smooth muscle cell proliferation, hirudin, and acetylsalicylic acid, amlodipine and doxazosin; anti-inflammatory agents such as glucocorticoids, betamethasone, dexamethasone, prednisolone, corticosterone, budesonide, estrogen, sulfasalazine, and mesalamine; antineoplastic/antiproliferative/anti-miotic agents such as paclitaxel, 5-fluorouracil, cisplatin, vinblastine, vincristine, epothilones, methotrexate, azathioprine, adriamycin and mutamycin; endostatin, angiostatin and thymidine kinase inhibitors, taxol and its analogs or derivatives; anesthetic agents such as lidocaine, bupivacaine, and ropivacaine; anti-coagulants such as D-Phe-Pro-Arg chloromethyl keton, an RGD peptide-containing compound, heparin, antithrombin compounds, platelet receptor antagonists, anti-thrombin antibodies, anti-platelet receptor antibodies, aspirin (aspirin is also classified as an analgesic, antipyretic and anti-inflammatory drug), dipyridamole, protamine, hirudin, prostaglandin inhibitors, platelet inhibitors and tick antiplatelet peptides; vascular cell growth promotors such as growth factors, Vascular Endothelial Growth Factors (VEGF, all types including VEGF-2), growth factor receptors, transcriptional activators, Insulin Growth Factor (IGF), Hepatocyte Growth Factor (HGF), and translational promotors; vascular cell growth inhibitors such as antiproliferative agents, growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; cholesterol-lowering agents, vasodilating agents, and agents which interfere with endogenous vasoactive mechanisms; anti-oxidants, such as probucol; antibiotic agents, such as penicillin, cefoxitin, oxacillin, tobranycin; angiogenic substances, such as acidic and basic fibrobrast growth factors, estrogen including estradiol (E2), estriol (E3) and 17-Beta Estradiol; and drugs for heart failure, such as digoxin, beta-blockers, angiotensin-converting enzyme (ACE) inhibitors including captopril and enalopril.

Some therapeutic materials include anti-proliferative drugs such as steroids, vitamins, and restenosis-inhibiting agents such as cladribine. Restenosis-inhibiting agents include microtubule stabilizing agents such as Taxol, paclitaxel, paclitaxel analogues, derivatives, and mixtures thereof. For example, derivatives suitable for use in the present invention include 2'-succinyl-taxol, 2'-succinyl-taxol triethanolamine, 2'-glutaryl-taxol, 2'-glutaryl-taxol triethanolamine salt, 2'-O-ester with N-(dimethylaminoethyl) glutamine, and 2'-O-ester with N-(dimethylaminoethyl) glutamide hydrochloride salt. Other therapeutic materials include nitroglycerin, nitrous oxides, antibiotics, aspirins, digitalis, and glycosides.

The support structure 22 may be formed from a bioabsorbable metal. A bioabsorbable metal is preferred because of its greater structural strength. Suitable bioabsorbable metals include known magnesium alloys, including formulations such as the magnesium alloy disclosed in U.S. Patent Application No. 2002/0004060 which includes approximately 50-98% magnesium, 0-40% lithium, 0-5% iron and less than 5% other metals. Other suitable formulations include a magnesium alloy having greater than 90% magnesium, 3.7%-5.5% yttrium, and 1.5%-4.4% rare earths, as disclosed in U.S. Patent Application No. 2004/0098108

Absorption inhibitor layer 24 and delivery layer 36 can also be formed of a variety of biocompatible materials. Such material may not be bioabsorbable.

Having described above various general principles, several exemplary embodiments of these concepts are now described. These embodiments are only exemplary, and many other combinations and formulations of support structure 22, absorption inhibitor layer 24, and delivery layer 36 formulations, and configurations are possible, contemplated by the principles of the invention, and will be apparent to the artisan in view of the general principles described above and the exemplary embodiments.

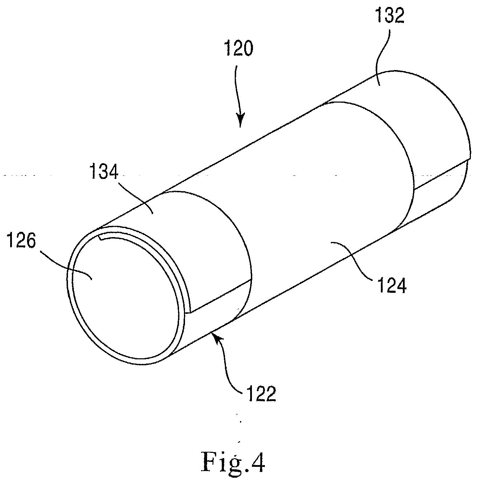

FIG. 4 illustrates a side perspective view of a stent 120 according to an embodiment of the invention. The stent 120 includes a support structure 122 formed of a bioabsorbable metallic or polymer material in a coil configuration. An absorption inhibitor layer 124 is disposed on a portion of an outer surface 130 of the support structure 122. In this embodiment, support structure 122 includes exposed portions 132 and 134. Exposed portions 132 and 134 of the support structure 122 will begin to be absorbed by the body in which the stent 120 is implanted immediately upon implantation, while the covered portions of support structure 122 will have a delayed absorption due to the absorption inhibitor layer 124.

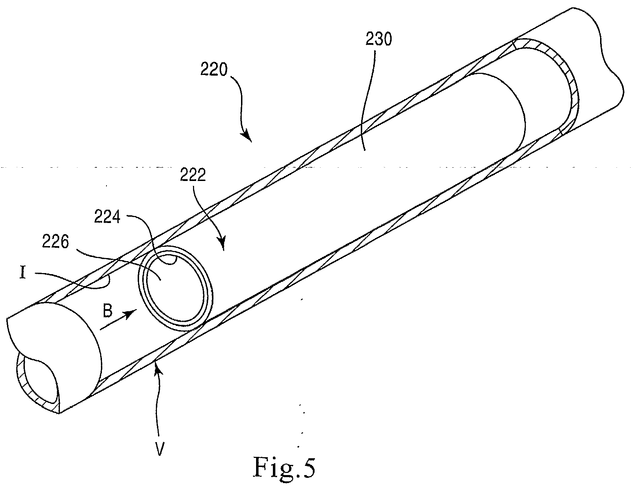

FIG. 5 illustrates a stent 220 according to another embodiment of the invention shown within a blood vessel V. Stent 220 is substantially tubular and includes a support structure 222 (the detailed configuration of which is omitted for ease of illustration), constructed of a bioabsorbable polymer or metallic material and an absorption inhibitor layer 224 at least partially disposed on an inner surface of the support structure 222. In this embodiment, an outer surface 230 of the support structure 222 will contact the blood vessel intimal layer I and have a first rate of absorption from contact with the intimal layer I, and the absorption inhibitor layer 24 will contact blood B flowing through the lumen of the blood vessel V.

In other embodiments, the absorption inhibitor layer 224 maybe disposed on the outer surface 230 of the support structure 222. In such an embodiment, the support structure 222 will contact the blood B flowing through the blood vessel V and the absorption inhibitor layer 224 will contact the intimal layer I of the blood vessel V.

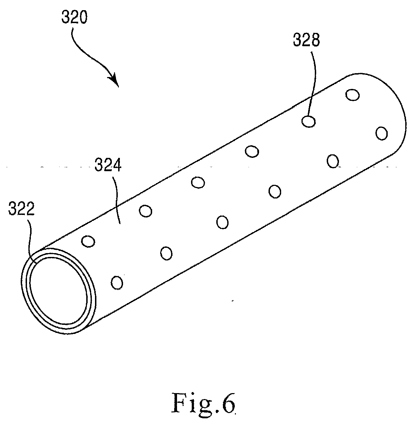

FIG. 6 illustrates a stent 320 according to another embodiment of the invention. In this embodiment, an absorption inhibitor layer 324 is disposed on an outer surface of a support structure 322. The absorption inhibitor layer 324 includes multiple pores 328. The pores 328 provide for a faster rate of absorption of that portion of support structure 322 where support structure 322 is exposed to the body lumen and/or bodily fluid through the pores 228.

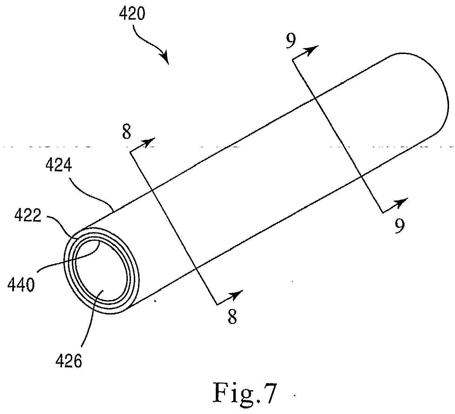

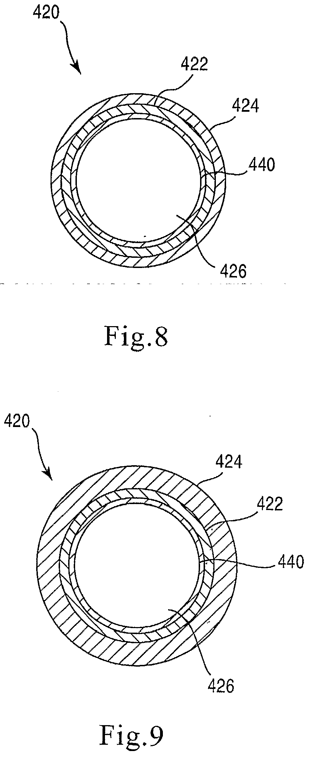

FIGS. 7, 8 and 9 illustrate a stent 420 according to yet another embodiment of the invention. Stent 420 includes a support structure 422 defining a lumen 426, and an absorption inhibitor layer 424 disposed on an outer surface of support structure 422. In this embodiment, the absorption inhibitor layer 424 is substantially overlying the outer surface of the support structure 422. Support structure 422 also includes a second absorption inhibitor layer 440 disposed on an inner surface of support structure 422. The absorption inhibitor layer 424 disposed on the outer surface of support structure 422 has varying thicknesses along the length of the support structure 422, as shown in the cross-sectional views of FIGS. 8 and 9. As stated previously, by varying the thickness of the absorption inhibitor layer 424, the rate of absorption of support structure 422 can be varied along the length of the support structure 422. In addition, because the support structure 422 includes both an absorption inhibitor layer 424 disposed on the outer surface, and an absorption inhibitor layer 440 disposed on the inner surface, the absorption of support structure 422 will be further delayed.

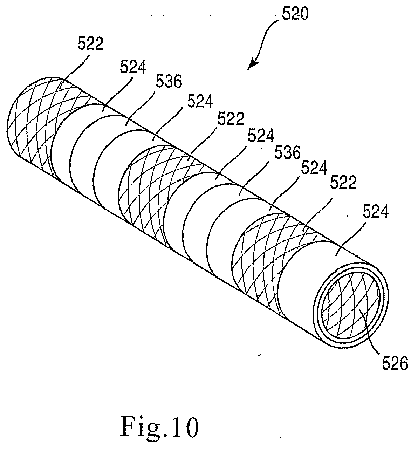

FIG. 10 illustrates a stent 520 according to another embodiment of the invention. The stent 520 includes a support structure 522 defining a lumen 526, and an absorption inhibitor layer 524 disposed in radial strips at spaced distances along the length of support structure 522. A delivery layer 536 is disposed in radial strips on an outer surface of two of the absorption inhibitor layers 524 in partially overlying relationship. The delivery layer 536 can contain a therapeutic agent configured to be released into the body as the delivery layer 536 is bioabsorbed into the body as previously described. Alternatively, a therapeutic agent may be contained in the absorption inhibitor layer 524. If the agent is contained in the absorption inhibitor layer 524, the release of the agent into the body will be delayed for those portions of the absorption inhibitor layer 524 that are covered with the delivery layer 536. To increase the rate of absorption in such an embodiment, the delivery layer 536 may be permeated, allowing the therapeutic agent to be released from the absorption inhibitor layer 524 and pass through the delivery layer 536 without waiting until the delivery layer 536 has begun to bioabsorb.

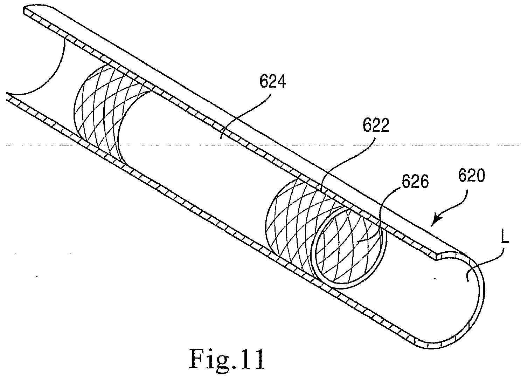

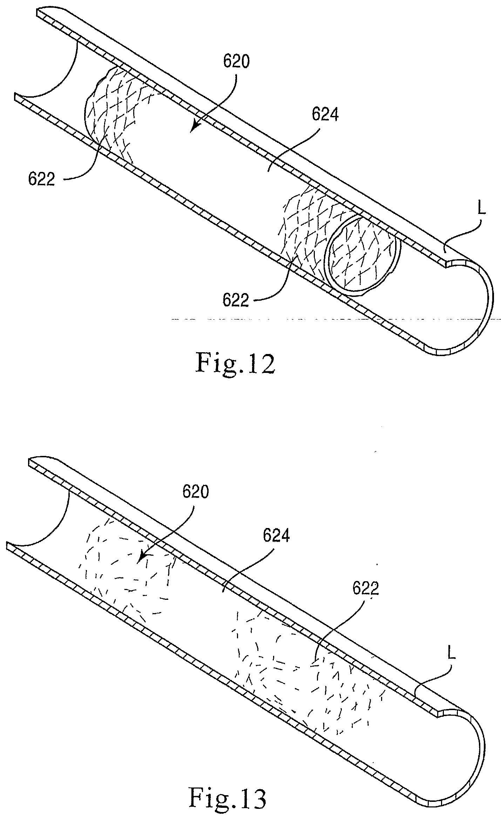

FIGS. 11, 12 and 13 illustrate a stent 620 shown positioned in a body lumen L at various stages of bioabsorption after insertion into the body lumen L. Stent 620 includes a support structure 622 defining a lumen 626, and an absorption inhibitor layer 624 disposed on a portion of an outer surface 630 of support structure 622. In this embodiment, support structure 622 is constructed with a metallic bioabsorbable material in a lattice framework configuration. FIG. 11 illustrates the stent 620 immediately after implantation into the body lumen, with no visible bioabsorption. FIG. 12 illustrates stent 620 with portions of support structure 622 bioabsorbed, while a substantial portion of the absorption inhibitor layer 624 still remains intact. FIG. 13 illustrates the stent 620 at a later time in the bioabsorption process from that shown in FIG. 12, where the support structure 622 is further bioabsorbed by the body lumen L, and the absorption inhibitor layer 624 is partially absorbed, exposing portions of the underlying surface of the support structure 622.

### Conclusion

While various embodiments of the invention have been described above, it should be understood that they have been presented by way of example only, and not limitation. Thus, the breadth and scope of the invention should not be limited by any of the above-described embodiments, but should be defined only in accordance with the following claims.

The previous description of the embodiments is provided to enable any person skilled in the art to make or use the invention.

The stent 20 (120, 220, 320, 420, 520, 620) may include more than one absorption inhibitor layer and one or more delivery layer. In some embodiments, the stent may include a therapeutic agent contained in one or more of the support structure, the absorption inhibitor layer and the delivery layer. The absorption inhibitor layer and/or the delivery layer may include pores and may be permeable to the therapeutic agent.

## Claims

1. A stent (20), comprising:
a support structure (22) formed of a first material, said first material being a metal that is absorbable by a mammalian body; and an absorption inhibitor (24) disposed on said support structure (22) in at least partially overlying relationship, said absorption inhibitor (24) being formed of a second material different from said first material, said second material being non-absorbable by the mammalian body, said absorption inhibitor (24) reducing a rate of absorption of said portion of said support structure (22) underlying said absorption inhibitor (24), wherein the support structure (22) has an inner surface (28) and an outer surface (30) and defines a lumen (26) **characterized in that** the absorption inhibitor (24) is alternatively disposed on the outer surface (30) or on the inner surface (28), wherein there is varying thickness of the absorption inhibitor (24) on a particular portion of the support structure (22).

2. The stent (20) of claim 1, wherein the absorption inhibitor (24) is a polymer.

3. The stent (20) of claim 1, wherein said support structure (22) is tubular shaped and configured to be disposed within a lumen of a blood vessel, said first material configured to contact the blood vessel intimal layer and has a first rate of absorption from contact with the intimal layer, said second material configured to contact blood flowing through the lumen of the blood vessel

4. The stent (20) of claim 1, wherein said absorption inhibitor (24) is permeable.

5. The stent (20) of claim 1, further including a therapeutic agent contained in at least one of said support structure or said absorption inhibitor (24), said therapeutic agent being absorbable by the mammalian body.

6. The stent (20) of claim 1, wherein said support structure (22) includes a coil configured to be disposed in a vascular lumen.

7. The stent (20) of claim 1, wherein said support structure (22) is tubular and configured to be inserted into a natural body lumen of a mammal.

8. The stent (20) of claim 1, wherein said support structure (22) has a first rate of absorption associated with said portion of said absorption inhibitor (24) having said first thickness and said support structure (22) has a second rate of absorption associated with said portion of said absorption inhibitor (24) having said second thickness, said first rate of absorption of said support structure (22) being different than said second rate of absorption of said support structure (22).

## Patentansprüche

1. Ein Stent (20) umfassend:
eine Stützstruktur (22) geformt aus einem ersten Material, wobei das erste Material ein Metall ist, das von einem Säugetierkörper aborbierbar ist; und ein Absorptionsinhibitor (24) der auf die Stützstruktur (22) in zumindest einer teilweise überlappenden Beziehung aufgebracht ist, wobei der Absorptionsinhibitor (24) aus einem zweiten Material geformt ist, das sich vom ersten Material unterscheidet, wobei das zweite Material von einem Säugetierkörper nicht absorbierbar ist, wobei der Absorptionsinhibitor (24) die Rate der Absorption des Teils der Stützstruktur (22), die unter dem Absorptionsinhibitor (24) liegt verringert, wobei die Stützstruktur (22) eine innere Oberfläche (28) und eine äußere Oberfläche (30) hat und ein Lumen (26) definiert, **dadurch gekennzeichnet, dass** der Absorptionsinhibitor (24) entweder auf der äußeren Oberfläche (30) oder auf der inneren Oberfläche (28) aufgebracht ist, wobei es eine variierende Dicke des Absorptionsinhibitors (24) auf einem bestimmten Teil der Stützstruktur (22) gibt.

2. Der Stent (20) nach Anspruch 1, wobei der Absorptionsinhibitor (24) ein Polymer ist.

3. Der Stent (20) nach Anspruch 1, wobei die Stützstruktur (22) eine tubuläre Form hat und ausgelegt ist in ein Lumen eines Blutgefäßes gebracht zu werden, wobei das erste Material ausgelegt ist, die innere Schicht der Blutgefäße zu kontaktieren und eine erste Absorptionsrate durch den Kontakt mit der inneren Schicht hat, wobei das zweite Material ausgelegt ist, Blut, das durch das Lumen des Blutgefäßes fließt, zu kontaktieren.

4. Der Stent (20) nach Anspruch 1, wobei der Absorptionsinhibitor (24) permeabel ist.

5. Der Stent (20) nach Anspruch 1, weiter umfassend einen therapeutischen Wirkstoff enthalten in zumindest der Stützstruktur oder dem Absorptionsinhibitor (24), wobei der therapeutische Wirkstoff von dem Säugetierkörper absorbiert werden kann.

6. Der Stent (20) nach Anspruch 1, wobei die Stützstruktur (22) einen Drahtring beinhaltet, der ausgelegt ist in ein Gefäßlumen eingebracht zu werden.

7. Der Stent (20) nach Anspruch 1, wobei die Stützstruktur tubulär ist und ausgelegt ist, in ein natürliches Körperlumen eines Säugetieres eingeführt zu werden.

8. Der Stent (20) nach Anspruch 1, wobei die Stützstruktur (22) eine erste Absorptionsrate hat, die mit dem Teil des Absorptionsinhibitors (24) assoziiert ist, der eine erste Dicke hat und wobei die Stützstruktur (22) eine zweite Absorptionsrate hat, die mit dem Teil des Absorptionsinhibitors (24) assoziiert ist, der eine zweite Dicke hat, wobei die erste Absorptionsrate der Stützstruktur (22) anders ist als die zweite Absorptionsrate der Stützstruktur (22).

## Revendications

1. Stent (20), comprenant :
une structure de support (22) constituée d'un premier matériau, ledit premier matériau étant un métal qui est absorbable par un organisme mammifère ; et un inhibiteur d'absorption (24) disposé sur ladite structure de support (22) selon une relation de superposition au moins partielle, ledit inhibiteur d'absorption (24) étant constitué d'un second matériau différent dudit premier matériau, ledit second matériau étant non absorbable par l'organisme mammifère, ledit inhibiteur d'absorption (24) réduisant une vitesse d'absorption de ladite partie de ladite structure de support (22) se trouvant en dessous dudit inhibiteur d'absorption (24), la structure de support (22) comportant une surface intérieure (28) et une surface extérieure (30) et définissant une lumière (26), **caractérisé en ce que** l'inhibiteur d'absorption (24) est disposé soit sur la surface extérieure (30), soit sur la surface intérieure (28), l'inhibiteur d'absorption (24) présentant une épaisseur variable sur une partie particulière de la structure de support (22).

2. Stent (20) selon la revendication 1, dans lequel l'inhibiteur d'absorption (24) est un polymère.

3. Stent (20) selon la revendication 1, dans lequel ladite structure de support (22) est de forme tubulaire et configurée pour être disposée à l'intérieur d'une lumière d'un vaisseau sanguin, ledit premier matériau étant configuré pour venir en contact avec la couche intimale du vaisseau sanguin et présentant une première vitesse d'absorption à partir du contact avec la couche intimale, ledit second matériau étant configuré pour venir en contact avec le sang s'écoulant à travers la lumière du vaisseau sanguin.

4. Stent (20) selon la revendication 1, dans lequel ledit inhibiteur d'absorption (24) est perméable.

5. Stent (20) selon la revendication 1, comprenant en outre un agent thérapeutique se trouvant dans ladite structure de support et/ou dans ledit inhibiteur d'absorption (24), ledit agent thérapeutique étant absorbable par l'organisme mammifère.

6. Stent (20) selon la revendication 1, dans lequel ladite structure de support (22) comprend un rouleau configuré pour être disposé dans une lumière vasculaire.

7. Stent (20) selon la revendication 1, dans lequel ladite structure de support (22) est tubulaire et configurée pour être insérée dans une lumière corporelle naturelle d'un mammifère.

8. Stent (20) selon la revendication 1, dans lequel ladite structure de support (22) présente une première vitesse d'absorption associée à ladite partie dudit inhibiteur d'absorption (24) présentant ladite première épaisseur et ladite structure de support (22) présente une seconde vitesse d'absorption associée avec ladite partie dudit inhibiteur d'absorption (24) présentant ladite seconde épaisseur, ladite première vitesse d'absorption de ladite structure de support (22) étant différente de ladite seconde vitesse d'absorption de ladite structure de support (22).
